# EUROPÄISCHE PATENTSCHRIFT

(11) **EP 0 934 520 B1**
(45) Veröffentlichungstag und Bekanntmachung des Hinweises auf die Patenterteilung: **21.09.2005**
(21) Anmeldenummer: 97913159.6
(22) Anmeldetag: 17.10.1997
(51) Int. Cl.: G01N 33/28, G01N 27/00, G01N 29/02, G01N 11/16

(54) **ÖLQUALITÄTSSENSOR**
OIL QUALITY SENSOR
DETECTEUR DE QUALITE D'HUILE

(30) Priorität: 26.10.1996 DE 19644572; 23.07.1997 DE 19731621
(43) Veröffentlichungstag der Anmeldung: 11.08.1999
(73) Patentinhaber: Volkswagen Aktiengesellschaft, 38436 Wolfsburg (DE)
(72) Erfinder: DICKERT, Franz, A-3400 Klosterneuburg (AT); FORTH, Peter, A-1200 Wien (AT); LIEBERZEIT, Peter, A-8650 Dornbirn (AT); VOIGT, Günter, D-38518 Gifhorn (DE); MARQUARDT, Klaus, Dieter, D-38446 Wolfsburg (DE)
(74) Vertreter: Fritz, Edmund Lothar
(86) Internationale Anmeldenummer: PCT/EP1997/005748
(87) Internationale Veröffentlichungsnummer: WO 1998/019156

(56) Entgegenhaltungen:
- WO-A-96/14573
- US-A- 4 741 200
- US-A- 5 151 110
- US-A- 5 201 215
- PATENT ABSTRACTS OF JAPAN vol. 95, no. 10, 30.November 1995 & JP 07 174685 A (HITACHI LTD.), 14.Juli 1995,
- PATENT ABSTRACTS OF JAPAN vol. 8, no. 257 (P-316) [1694] , 24.November 1984 & JP 59 126931 A (NIPPON JIDOSHA BUHIN SOGO KENKYUSHO K.K.), 21.Juli 1984,

## Beschreibung

Die Erfindung betrifft einen Ölqualitätssensor gemäß dem Oberbegriff des Anspruchs 12, ein Verfahren zur Feststellung eines Analyten und die Verwendung eines Sensors.

Motorenöle haben sich im Laufe der Zeit zu High-Tech-Produkten entwickelt, die mit Voraussetzung sind, hohe Motorleistungen zur ermöglichen. "Motorenöle" stellen dabei eine Sammelbezeichnung für Grundölkomponenten aus Mineralöl, Hydrocrackanten und synthetischen Komponenten dar. Motorenöle enthalten noch Additive, die als Fertigmischung ("Paket") zugegeben werden, sowie Viskositätsindex-Verbesserer (VI). Die Motorenöle dienen dabei als Schmierstoffe für die Motoren sowie als Kühl- und Abdichtmedium. Weiterhin sollen sie sämtliche Motorenteile reinigen und reinhalten. Die VI-Verbesserer bewirken ein günstigeres Viskositäts-Temperaturverhalten, als es die reinen Grundöle aufweisen. Der Anteil an Additiven und VI-Verbesserer liegt je nach Anforderungen, die an das Öl gestellt werden, üblicherweise zwischen 5 und 25 %.

Weitere Aufgaben der Additive in den Motorenölen sind die Verbesserung der Korrosionsschutzeigenschaften der Öle sowie die Verhinderung von Schlammablagerungen und Öleindickungen sowie der Verschleißschutz an den Reibpartnern unter allen auftretenden Belastungen. Die thermischen Beanspruchungen der Motorenöle sind hoch und liegen dabei im Ölsumpf durchschnittlich bei ca. 100 bis 150 °C. Im Bereich der oberen Kolbenringzone können Temperaturspitzen zwischen 200 und 350 °C auftreten.

Im Laufe ihres Gebrauchs altern die Öle, wobei in erster Linie die Additivkomponenten und die VI-Verbesserer abgebaut (verbraucht) werden. Einen erheblichen Anteil an der Ölalterung haben unverbrauchte, teilweise oxidierte und polymerisierte Kraftstoffkomponenten. Somit wird die Ölalterung durch Temperatureinwirkung und reaktionsfähige Verbrennungsprodukte (Radikale) sowie über das Überschreiten der Dispergierfähigkeit der Öle für Feststoffe und Alterungsprodukte hervorgerufen. Hierdurch werden dann die notwendigen Eigenschaften der Öle zum störungsfreien Betrieb der Motoren mitunter drastisch verschlechtert. Eine erhöhte Viskosität hat z. B. beim Startvorgang einen verlängerten Transport des Öles zu den Schmierstellen zur Folge, wodurch ein Anstieg des Verschleißes eintritt.

Der Verbrauch der Dispergieradditive hat eine Verschlechterung der Fähigkeit der Öle zur Reinhaltung der Motoren, insbesondere an kritischen Schmierstellen, wie im Bereich der Kolbenringe/Nuten und Feuerstege, zur Folge, sowie eine Verschlechterung bei der Verhinderung der Ablagerungsbildung an Ventilen und im Ventiltrieb.

Wünschenswert ist es daher, die während des motorischen Betriebes zwangsläufig auftretende Verschlechterung der Eigenschaften der Motorenöle kontinuierlich oder in kurzen Zeitabschnitten, d. h. beispielsweise ein oder mehrmals während eines Betriebes einer Brennkraftmaschine, zu erfassen.

Bis heute ist es jedoch noch nicht gelungen, zuverlässige Meßfühler für eine Ölzustandsanalyse zu entwickeln, wobei für einen längeren Betrieb des Motorenöls im Motor, insbesondere bei instationären Motoren, eine on-board-Analyse, d. h. eine Analyse direkt am Motor, erforderlich ist.

Bisher wurden die unterschiedlichsten Ölsensoren entwickelt, die insbesondere die Viskosität, TAN (Total Acid Number) oder den Füllstand sensieren. Eine besondere Schwierigkeit ist hierbei die Verwendung unterschiedlicher Öle an ein und derselben Brennkraftmaschine sowie das Kompensieren unterschiedlicher Alterungseinflüsse auf die sensierte Eigenschaft. So ist es beispielsweise aus US-A 4,675,662 und 4,721,874, EP 527 176 B und JPN. Appl. Phys., 1993, Acoustic Plate Viskosity Sensor, bekannt, die sich mit der Alterung des Öls geänderte Viskosität als Meßgröße für den Ölzustand heranzuziehen. Dies geschieht über akustische Laufzeitveränderungen, die Phasenverschiebung oder über Eigenfrequenzänderungen eines Schwingquarzes. Problematisch sind hierbei zum einen die häufig fehlende Möglichkeit, die Messung on-board vorzunehmen, und zum anderen die möglichen gegenläufigen Effekte "Abbau des Motorenöls und Verdünnung durch Kraftstoff", die die Viskosität erniedrigen, gegenüber der "Verknüpfung der Abbauprodukte", die die Viskosität erhöhen, solange sie nicht als Schlamm ausfallen.

Die TAN oder auch TBN (Total Basic Number) ist vom Grundprinzip für ein on-board-Messung nicht geeignet, da hierbei das Altöl mit KOH titriert wird. Neuere Ansätze, wie beispielsweise aus SAE 910497, SAE 962112, US-A 4,675,662, 4,792,791 und 5,200,027 bekannt, zeigen hier interessante Lösungen, die beispielsweise mit Kapazitätssensoren, Messung der lonenwanderung oder einer Potentialdifferenz, mit elektrochemischen Festkörperzellen und mit Korrosionssensoren arbeiten. Diese Lösungsansätze sind teils ungenau, noch zu groß und zu schwer oder benötigen ein Opferbauteil, das grundsätzlich unerwünscht ist: Ferner sind noch mathematische Modelle (SAE 870403) und HC-Abgassensoren (DE 42 35 225) bekannt, die bislang auch noch nicht zu einem Durchbruch geführt haben. Auch Füllstandssensoren sind wenig geeignet, da diese beispielsweise bei einer starken Verdünnung des Motorenöls durch Kraftstoffe versagen.

In "Molekular Imprinting of Chemically Sensitive Coatings - New Strategies for Sensor Design and Fabrication - " von F. L. Dickert, P. Forth, P. Lieberzeit, M. Tortschanoff, W.-E. Bulst, U. Knauer und G. Fischerauer in "Sensor 97" 8. internationale Messe, Nürnberg, 1997, wird ein molekulares Prägen für massensensitive Sensorik in Gasen wie in Flüssigkeiten näher beschrieben.

US-A-5,151,110 beschreibt einen selektiven chemischen Sensor, der aus einem piezoelektrischen Bestandteil und einer Beschichtung dieses Bestandteiles besteht. Diese Beschichtung besitzt eine poröse Struktur, die spezifische chemische Moleküle anhand ihrer Größe aus einer flüssigen Probe zu adsorbieren vermag. Die daraus resultierende Gewichtsveränderung des Sensors bewirkt eine veränderte Schwingungsfrequenz, wodurch ein Analyt bestimmt werden kann.

US-A 5,201,215 beschreibt einen mikrochemischen QMB (quartz crystal microbalance) Sensor, der zur gleichzeitigen Messung von Gewichts- und Viskositätsänderungen einer Flüssigkeit benutzt werden Kann. Diese Messungen können auch unabhängig voneinander durchgeführt werden. Der piezoelektrische Bestandteil des Sensors ist von einer Schicht umgeben, die aus einem Polymer bestehen kann. An diese Schicht binden spezifische Analyte einer flüssigen Probe, wodurch es zu einer Gewichtsveränderung des Sensors kommt.

Aufgabe der vorliegenden Erfindung ist es, einen Sensor zur Verfügung zu stellen, der auch on-board an einer Brennkraftmaschine Messungen vornehmen kann, wobei der Sensor weitgehend zuverlässig und störungsfrei die Alterung eines Öls bestimmen soll.

Die Lösung dieser Aufgabe liefert ein Ölqualitätssensor mit den Merkmalen des Anspruchs 12.

Gegenstand der Erfindung ist weiterhin die Verwendung eines Sensors mit einer sensitiven Schicht zur Feststellung eines Analysten in einem Öl und/oder zur Feststellung eines öligen Analyten gemäß Anspruch 1 sowie ein Verfahren zur Feststellung eines Analyten in einer flüssigen Phase mittels eines Sensors, bei dem die Alterung eines Öls bestimmt wird gemäß Anspruch 15.

Die Unteransprüche zeigen bevorzugte Ausführungsformen, mit denen sich besonders günstige Sensoren, Verwendungen von Sensoren sowie Ölqualitätsermittlungen durchführen lassen.

Wenn im folgenden auf eine Schicht, eine Oberfläche, ein Volumen oder ein Material Bezug genommen wird, gilt dieser Bezug sinngemäß für die ganze Gruppe sowie für alle Verwendungen, Verfahren und Sensoren dieser Erfindung. Bei der Schicht handelt es sich um ein mit einem Substrat fest verbundenes Material.

Die erfindungsgemäße Feststellung des Analyten ist in der Regel nicht nur qualitativ, sondern insbesondere zumindest halbquantitativ. Das heißt, mit der Feststellung des Analyten erfolgt auch eine genaue bzw. geschätzte Konzentrations- bzw. Mengenbestimmung des Analyten. Bei einer qualitativen Feststellung kann das Erreichen des Feststellungsschwellwertes ggf. in Verbindung mit einer mathematischen Methode (z. B. Zeitablauf) zur Signalisierung (des Ölwechsels) herangezogen werden.

Prinzipiell ist erfindungsgemäß der Sensor in allen Flüssigkeiten einsetzbar, in denen eine stoffliche Veränderung der Zusammensetzung erfolgt, d. h. in denen mindestens eine Komponente zu- bzw. abnimmt. Bevorzugt wird der Sensor zur Charakterisierung einer komplexen Flüssigkeit eingesetzt, d. h. in einer Flüssigkeit, die mehrere von der Struktur und Menge her unbekannten Komponenten enthält, wobei insbesondere eine Wiederholbarkeit der exakten Zusammensetzung der Flüssigkeit meist nicht gegeben ist, z. B. weil die Zusammensetzung der Flüssigkeit durch eine (nicht überschaubare) Vielfalt von Einflüssen bedingt ist. Ganz besonders vorteilhaft kommt der Sensor in einer ölhaltigen Flüssigkeit zum Einsatz, bevorzugt in einer Flüssigkeit die mindestens zu 30% und insbesondere zu mindestens 50 % ein Öl enthält. Neben dem Öl können noch verschiedene andere Komponenten vorliegen, die ggf. auch von dem Sensor erkannt werden können. Bevorzugt ist der Sensor ausgelegt auf einen oder mehrere Bestandteile des Öls, der/die im Laufe des Gebrauchs des Öls (bzw. der Flüssigkeit) abnehmen; es ist jedoch auch eine Auslegung des Sensors möglich auf eine Zunahme eines oder mehrere Bestandteile der Flüssigkeit. Eine solche Zunahme liegt z. B. bei der steigenden Acidität mit der Alterung des Öls vor, d. h., es kann auch die Zunahme der aciden Verbindungen festgestellt werden. Vorteilhaft ist der Sensor ausgelegt auf eine oder mehrere Hauptkomponenten der Flüssigkeit, d. h. solche Komponenten, die den Hauptgewichtsanteil der Flüssigkeit, beispielsweise der Ölzusammensetzung, ausmachen.

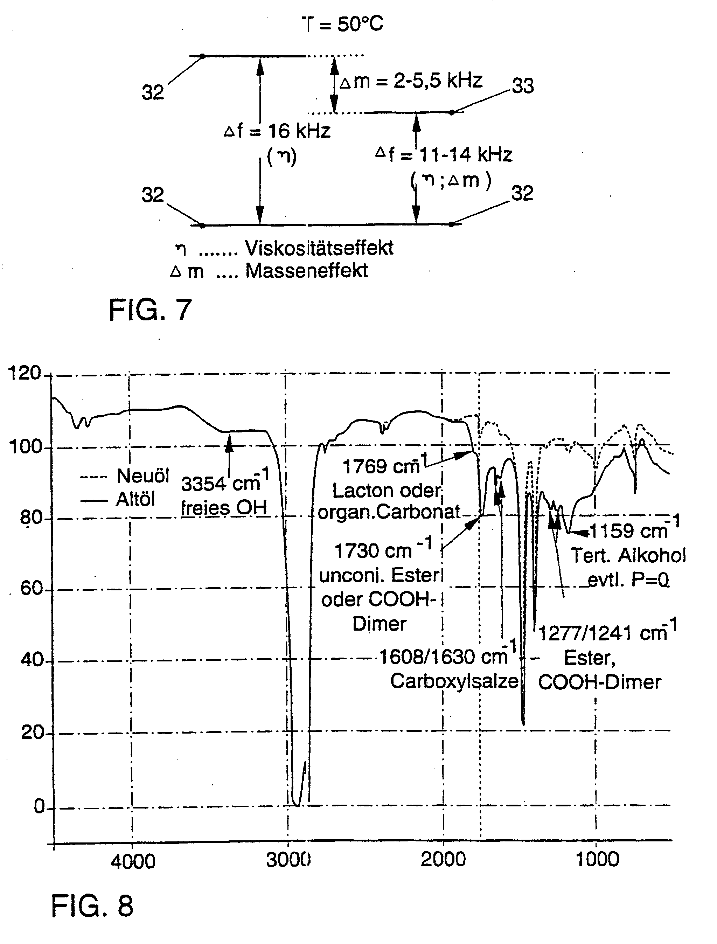

Die oben beschrieben aciden Veränderungen bei der Alterung eines Motorenöles sind IRspekroskopisch nachweisbar. So zeigt das Altöl-IR-Spektrum in Figur 8 gegenüber dem Neuöl-IR-Spektrurn deutlich Absorptionsbanden der Hydroxylgruppen bei 3 353 cm⁻¹, die von Alkoholen bzw. organischen Säuren herrühren. Weiterhin enthält das Altölspektrum eine Alkoholbande bei 1 159 cm⁻¹ (C-C-O Gerüstschwingungen für Alkohole, insbesondere tertiäre bzw. senkundäre - könnte aber auch eine P=O Streckschwingung sein) und Säurebanden bei 1730 cm⁻¹ (C=O Bande) und 1 277 bzw. 1241 cm⁻¹ (-C-O Streckschwingungen). (Literaturzitat: The Handbook of Infrared and Raman Characteristic Frequencies of Organic Molecules - Daimay Lien-Vien et al., Academic Press 1991.) Besonders deutlich sind die aciden Veränderungen im Öl IR-spektrpskopisch über die intensiven C=O-Banden von Carboxylat- bzw. Carboxylgruppierungen, wie sie bei 1 608/1 630/1 730/1 769 cm⁻¹ zu erkennen sind, nachweisbar.

Im Gegensatz zu den eingangs beschriebenen Sensoren wird bei der erfindungsgemäßen Messung ein Sensor verwendet, der mindestens einen Bestandteil der Flüssigkeit wiederholt ein- und auslagern kann, vorteilhaft entsprechend der Konzentration dieses Bestandteils.

Gegenüber bekannten Sensoren, wie z. B. Glaselektroden, unterscheidet sich der erfindungsgemäß eingesetzte Sensor durch mindestens eines der folgenden Merkmale:
- Der Bestandteil der Flüssigkeit hat ein Molekulargewicht ≥ 150, insbesondere ≥ 200;
- der Bestandteil der Flüssigkeit wind am Sensor im wesentlichen absorbiert bzw. adsorbiert;
- der (ermittelte) Bestandteil der Flüssigkeit ist nicht kationisch, insbesondere nicht ionisch;
- es wird nicht ein Grenzflächenpotential, elektrochemisches Potential und/oder Wasserstoffpotential als charakteristische Größe bestimmt.

Der Bestandteil ist dabei so gewählt, daß er sich im Laufe des Gebrauchs der Flüssigkeit hinsichtlich seiner Affinität zu der sensitiven Schicht verändert, üblicherweise wird dies erreicht durch eine Konzentrationsänderung des Stoffes in der Flüssigkeit durch den Gebrauch derselben.

Erfindungsgemäß kann besonders vorteilhaft ein (bzw. mehrere) Bestandteil der Flüssigkeit dadurch gemessen werden, daß der Bestandteil sich in die sensitive Schicht einlagert bzw. sich aus dieser herauslöst, wobei eine Gewichtsveränderung der sensitiven Schicht stattfindet. Die Einlagerung kann auch als Volumeneffekt bezeichnet werden, d. h. die Einlagerung des oder der Analyten geschieht zumindest bis zu einer bestimmten Schichtdicke im wesentlichen proportional der Schichtdicke. Durch Messung der Gewichtsveränderung erhält man ein Maß für die Einlagerung des/der fraglichen Bestandteils/e und hierdurch ein Maß für dessen/deren Konzentration. Zusätzlich oder alternativ wird gemäß der vorliegenden Erfindung ein (bzw. mehrere) Analyt (ein Bestandteil) in einem Öl und/oder ein (bzw. mehrere) öliger Analyt durch die Einlagerung des (bzw. der) Analyten in die sensitive Schicht bestimmt.

Als Analyt und/oder Öl eignet sich insbesondere ein Motorenöl, wie es beispielsweise in Brennkraftmaschinen zum Einsatz kommt. Vorteilhaft ist der Analyt hierbei zumindest ein Bestandteil eines neuen Motorenöls, er kann jedoch auch ein Bestandteil des bei der Benutzung des Motorenöls sich bildenden Altöls sein, insbesondere ein Oxidationsprodukt. Bevorzugt hat der Analyt hierbei wiederum einen aliphatischen Rest, wie er üblicherweise in mineralischen und/oder synthetischen Motorenölen vorliegt. Solche Kohlenwasserstoffreste haben üblicherweise ein Molekulargewicht zwischen 300 und 3 000. Durch den erfindungsgemäßen Einsatz der adaptierten sensitiven Schicht kann z. B. erreicht werden, daß sich beispielsweise entweder im wesentlichen nur die Neuölkomponenten in die Schicht einlagern, oder im wesentlichen nur die Abbauprodukte (mit Keto-, Aldehyd- und/oder Säureresten bzw. deren Additions- und/oder Kondensationsprodukte).

Besonders vorteilhaft ist die sensitive Schicht des Sensors aus einem Polymer aufgebaut. Dies ermöglicht eine einfache und kostengünstige Herstellung und leichte Adaption an einen Analyten. Als Polymer eignen sich insbesondere Polyurethane und/oder modifizierte Polyurethane, beispielsweise solche, bei denen die OH-Komponente zumindest teilweise gegen eine NH₂-Komponente ausgetauscht ist. Für den Nachweis der Abbauprodukte, insbesondere acider Veränderungen des Öls, ist es vorteilhaft, wenn in die Polymerstruktur basische Komponenten eingebaut werden. Dies kann beispielsweise durch den Einsatz basischer Monomerer bzw. von Prepolymeren mit basischen Resten erfolgen, wobei die Basizität vorteilhaft bei der Polymerbildung erhalten bleibt. Ein solches basisches Monomer ist beispielsweise Triethanolamin N(CN₂CH₂OH)₃, das als Vernetzer wirkt und dessen tertiäre Amingruppe bei der Polymerisation zum Polyurethan erhalten bleibt. Bei einer solchen Polymerisation erfolgt die Adaption an Altöl, d. h. die Polymerisation geschieht in Gegenwart von Altöl, das sich anschließend aus dem fertigen Polymer wieder herauslösen läßt. Der im Triethanolamin enthaltene Stickstoff kann anschließend polare Wechselwirkungen zu den aciden Komponenten im Altöl eingehen und somit diese Komponenten aus einem Gemisch, auch in Gegenwart von gewisser Mengen an unverbrauchtem Öl, extrahieren. Zudem bietet das Triethanolamin in den so erhaltenen Schichten ganz allgemein polare Wechselwirkungszentren für Analytmoleküle mit adaptierten Eigenschaften an.

Die Adaption an den Analyten kann gemäß einer Alternative geschehen, indem die Schicht zusammen mit dem Analyt gebildet wird, beispielsweise durch Mischen eines Motorenöls mit dem Schichtbildner, insbesondere einem Monomer oder Prepolymer. Bevorzugt wird die Schichtzusammensetzung derart gewählt, daß der Analyt wie auch die gesamte Flüssigkeit inert zu der Schicht ist, d. h. diese weder abbaut noch sonstwie chemisch verändert.

Weiterhin wird erfindungsgemäß der Sensor vorteilhaft zusammen mit einem nicht-sensitiven Sensor (Meßelement gleichen Aufbaus wie der Sensor, jedoch ohne die sensitiven Eigenschaften für einen Bestandteil der Flüssigkeit) eingesetzt, der als Referenz dient. Dies ermöglicht einen einfachen und sicheren Meßaufbau. Ganz besonders vorteilhaft können auch unterschiedlich sensitive Sensoren gemeinsam, insbesondere auch in Verbindung mit einem nicht sensitiven Sensor, eingesetzt werden. Hierbei ist dann ein Sensor auf das Erkennen von Neuöl, und der andere Sensor auf das Erkennen von Altöl ausgelegt. Diese unterschiedlich sensitiven Sensoren können sich gegenseitig als Referenz dienen, so daß Viskositätsveränderungen ausgemittelt werden können. Wie erwähnt, kann jedoch besonders vorteilhaft ein dritter, nicht sensitiver Sensor eingesetzt werden, über den eine Viskositätsveränderung ermittelt wird. Mit solchen Kombinationen lassen sich die verschiedensten Ölveränderungen besonders zuverlässig erkennen, so daß der Ölsensor für die unterschiedlichsten Öle (bzw. andere Flüssigkeiten) eingesetzt werden kann. Durch den Einsatz mehrerer (unterschiedlich) sensitiver Sensoren kann außerdem auch der Ausfall eines Sensors bzw. eine mögliche verringerte Empfindlichkeit eines Sensors, wie sie beim Einsatz der unterschiedlichsten Öle vorkommen kann, erkannt werden, wodurch eine besonders hohe Prozeßsicherheit gegeben ist.

Die Messung selbst erfolgt vorzugsweise durch Versetzen der Schicht in Schwingungen, auch eine dielektrische Auswirkung der Schicht kann beispielsweise als Meßprinzip herangezogen werden. Bei dem Versetzen der Schicht in Schwingungen wird die Schicht vorteilhaft auf einen Schwingkristall (Quarzkristall) aufgebracht, der dann zu Schwingungen angeregt wird. Durch die Beladung der sensitiven Schicht mit dem Analyt wird die Schicht entsprechend der Beladungsmenge schwerer, wodurch sich das Schwingungsverhalten verändert. Hieraus kann wiederum auf die Beladungsmenge zurückgeschlossen werden.

Zusätzlich zu der Referenz, über die die absolute Viskosität (bzw. eine Viskositätsveränderung) ermittelt werden kann, kommt vorteilhaft noch ein Temperatursensor zum Einsatz, da die Viskosität (insbesondere bei Motoröl) stark temperaturabhängig ist.

Das temperaturabhängige Viskositätsverhalten der flüssigen Phase kann hierbei beispielsweise in einem Kennfeld abgelegt sein, über das dann die Temperaturkorrektur der Meßwerte erfolgt. Alternativ oder zusätzlich kann die Messung auch nur bei einer oder mehreren festgelegten Temperaturen durchgeführt werden.

Wie bereits erwähnt, können mehrere Sensoren parallel eingesetzt werden, die auf unterschiedliche Bestandteile der flüssigen Phase und/oder unterschiedliche flüssige Phasen geprägt sind. Hierdurch lassen sich unterschiedliche Veränderungen der flüssigen Phase erkennen. Dies ist insbesondere beim Einsatz des Sensors in einem Motoröl von Bedeutung, da hier zum einen unterschiedliche Öle mit unterschiedlichen Bestandteilen eingesetzt werden können, und zum anderen - je nach den Betriebsbedingungen des Motaröls - unterschiedliche Alterungsprozesse ablaufen können.

Zum Einsatz des Sensors gehört ferner eine Auswerteelektronik, über die der Sensor einerseits betrieben und andererseits das Sensorsignal zu der gewünschten Information umgewandelt wird. Bei Motoröl kann die Information insbesondere ein Hinweis auf die Fälligkeit des Motorölwechsels sein.

Grundsätzlich kann für die vorliegende Erfindung praktisch jede Schicht eingesetzt werden, die eine Matrix mit Cavitäten und/oder Diffusionskanälen enthält, die an einen zu bestimmenden Bestandteil der Flüssigkeit (Analyt) angepaßt (adaptiert) sind, d. h. den Analyt entsprechend seiner Konzentration einlagern (bei einer hohen Konzentration) bzw. auslagern (bei einer niedrigen Konzentration des Analyten in der Flüssigkeit).

Das Herstellen solcher adaptierter Oberflächen ist auch als "molekulares Prägen" bekannt, wobei solche molekular geprägten Schichten im wesentlichen nur in Gasen eingesetzt wurden. Solche molekular geprägten Schichten lassen sich industriell und kostengünstig herstellen. Bei der Generierung der Sensorschicht, beispielsweise durch einen Polymerisationsprozeß, wird der zu detektierende Analyt der Reaktionsmischung zugesetzt. Die Analytmoleküle hinterlassen bei der Polymerisation bzw. der Aushärtung ihren Abdruck in der Matrix und können nach Abschluß der Reaktion verdampft oder aus dem (polymeren) Netzwerk herausgespült werden. Hierzu ist ein bezüglich des Polymerisationsprozesses und der fertigen polymeren Schicht inerter Analyt zu wählen (bzw. die Reaktionsmischung ist entsprechend auszuwählen). Die Qualität der molekular geprägten Schichten hängt dabei von vielen Einflüssen ab, insbesondere von der Schichtmaterialwahl (Polymer), Polymerisationsdauer, Lösemittelanteil bei der Schichtherstellung, Temperatur, Vernetzeranteil etc. Durch diesen Herstellungsprozeß verbleiben in der chemisch-sensitiven Schicht an den Analyt adaptierte Cavitälen und Diffusionskanäle, die zur Wiedereintagerung des Analyten prädestiniert sind. Durch diese Prägung kommt es analog zum Schlüssel-Schloß-Prinzip zu analyt-spezifischen Wechselwirkungen, entsprechend der Konzentration des Analyten. Dieser Prägeprozeß kann nicht nur mit einem chemisch-reinen Analyten, sondern auch mit einem Analytgemisch complexer Zusammensetzung durchgeführt werden. Somit ist auch die Charakterisierung der vielfältigen Alterungsprozesse bei Motorenölen mit so hergestellten Sensoren möglich. Erfindungsgemäß kann also eine solche sensitive Schicht beispielsweise durch Polymerisation eines Polyurethans mit einem bestimmten Anteil an Vernetzermolekülen in Gegenwart von z. B. frischem Motorenöl (beispielsweise für Otto-Motoren; auf Mineralölbasis und/oder synthetisch) hergestellt werden. Nach dem Ausspülen des Öls aus der polymerisierten Schicht bleiben in der polymeren Matrix den Bestandteilen des frischen Motorenöls angepaßte Hohlräume zurück. Mit Hilfe der Infrarotspektroskopie kann gezeigt werden, daß diese Hohlräume bemerkenswert selektiv Neuöl reinkorporieren. Entsprechendes gilt auch für die Prägung mit Altöl, wobei bei der Prägung mit Altöl die Selektivität geringer ist, d. h., es werden auch geringe Mengen an Neuöl eingelagert, wodurch die Response (z. B. Gewichtsveränderung; elektronisches Signal) geringer ist.

Die Erfindung wird im folgenden anhand von Zeichnungen und Ausführungsbeispielen näher beschrieben.

Es zeigen:
- Figur 1: IR-Spektren von Otto-Neuöl und Otto-Altöl in Tetrachlorkohlenstoff, CH₂/CH₃ Streckschwingungen;
- Figur 2: IR-Spektren einer Non-Imprint-Probe nach verschiedenen Arbeitsschritten, CH₂/CH₃/OH Streckschwingungen;
- Figur 3: IR-Spektren eine Imprint-Probe nach den einzelnen Arbeitsschritten;
- Figur 4: IR-Spektren einer Imprint-Probe eines aminhaltigen Polymeren nach verschiedenen Arbeitsschritten;
- Figur 5: die Temperaturabhängigkeit der Frequenzantwort eines unbeschichteten QMB in Alt und Neuöl;
- Figur 6: die Frequenzantwort eines mit Neuöl geprägten QMB beim Wechsel von Neuöl auf Altöl;
- Figur 7: einen Vergleich der Frequenzantworten massensensitiv und nichtmassensensitiv beschichteter QMB;
- Figur 8: IR-Spektren von frischem und gebrauchtem Otto-Motoröl; und
- Figur 9: einen Vergleich der Frequenzantworten Neuöl- und Altöl-geprägter QMB.

### Experimentelles:

### Präparation der Schichten

Es werden Sensorschichten zur Bestimmung der Alterung von Motorenöl hergestellt. Die chemisch sensitiven Schichten werden durch molekulares Prägen von verschiedenen Polyurethanen hergestellt. Folgende Chemikalien werden verwendet:
- Phloroglucin puriss;
- Bisphenol A, 97 %;
- Triethanolamin p. A;
- Diphenylmethandiisocyanat z. Synt (Gemisch aus 70% Diisocyanat und 30 % Triisocyanat);
- Hexamethylendiisocyanat z. Synt.;
- Tetrahydrofuran getrocknet p. A..

Jedes der synthetisierten Polyurethane enthält im stöchiometrischen Verhältnis eines der Isocyanate und eine Mischung der beiden alkoholischen Komponenten. Der Anteil des Phloroglucins in der alkoholischen Kc mponente bestimmt den Vernetzungsgrad. Ein günstiger Vemetzungsgrad für Imprints (für die Wiedereinlagerung des Analyten) liegt bei ca. 60 % (je nach Polymer allg. 20 - 85 %). Bei Verwendung des Hexamethylendiisocyanats setzt man hierfür ein Gemisch aus 60 mal-% Phloroglucin und 40 mol-% Bisphenol A ein; für das Isocyanatgemisch liegt der Anteil des Phloroglucins im Alkoholgemisch bei 40 mol-%, da hier auch das Isocyanat aufgrund seines Triisocyanatanteils als Vernetzer wirkt.

Zur Herstellung der Schichten werden die einzelnen Komponenten zusammen mit der geeigneten Menge Neuöl in THF gelöst. Diese Mischung kann direkt auf Glasplättchen oder Quarzplättchen zur Vermessung mittels FT-IR oder auf Schwingquarze aufgebracht werden.

### 1.) Hexamethylendiisocyanat:

0,76 mg (6 mmol) Phloroglucin, 91 mg (4 mmol) Bisphenol A, 218 mg (13 mmol) Hexamethylendiisocyanat und 116 mg Otto-Neuöl (entspricht 30 % der Masse des Monomerengemisches und somit 23,1 % Anteil an der Gesamtmasse der Matrix) werden in 1 ml THF gelöst.

Von diesem Ausgangsgemisch werden 20 µl mit 180 µl THF verdünnt und 20 µl der Verdünnung auf das Glasplättchen bzw. 2 µl auf den QMB aufgebracht.

### 2.) Diphenylmethandiisocyanat:

50 mg Phloroglucin (4 mmol), 137 mg Bisphenol A (6 mmol), 274 mg Diphenylmethandiisocyanat (10,4 mmol) werden zusammen mit 138 mg Öl in 1 ml THF gelöst. Verdünnung und Auftragung analog zur obigen Schicht.

### 3.) Triethanolamin:

Von den einzelnen Komponenten wurden Lösungen der Konzentration 2.5 mmol/2ml in getrocknetem THF hergestellt; dies bedeutet folgende Einwaagen:

| | |
|---|---|
| Diphenylmethandiisocyanat | 657 mg |
| Phloroglucin | 315 mg |
| Bisphenol A | 570 mg |
| Triethanolamin | 373 mg |

Otto-Altöl wurde in Eppendorfgefäßen eingewogen (62.8 mg bei 30 % Ölgehalt in der Schicht, 48,9 mg für 25 % und 36,7 mg für 20 %) und in 0,5 ml THF gelöst.

Dieser Lösung wurden dann zuerst die alkoholischen Komponenten mit Hilfe einer Gilsonpipette zugefügt und erst kurz vor dem Auftragen das Isocyanat. Die Mischungen setzen sich wie folgt zusammen:

| | |
|---|---|
| Bisphenol A | 148 µl Lösung |
| trifunktioneller Alkohol | 100 µl Lösung |
| Isocyanat | 231 µl Lösung |

Die beiden trifunktionellen Komponenten Phloroglucin und Triethanolamin wurden im benötigten Verhältnis gemischt (z. B. 10 % TEA in der Sicht → 10 µl TEA-Lösung und 90 µl Phloroglucinlösung).

Die besten Altöl-Wiedereinlagerungen werden mit 10 % und 20 % TEA gefunden.

### FT-IR-Messungen

Die über Nacht auspolymerisierten Plättchen werden in einem FT-IR Gerät (Perkin-Elmer FTIR 2000) gegen ein leeres Quarzplättchen als Blank vermessen. Zur Interpretation werden die symmetrische sowie die antisymmetrische Methylenschwingung bei 2 856 cm⁻¹ und 2 921 cm⁻¹ herangezogen.

Die wesentlichen Meßpunkte erhält man nach folgenden Arbeitsschritten:
1.) Auspolymerisieren.
2.) Ausspülen des Motoröls aus der Schicht durch Rühren in n-Heptan.
3.) Wiedereinlagerung des Neuöls (1 Nacht in Öl einlegen, rühren). Zum Entfernen des oberflächlichen Öls wird das Plättchen zuerst mit Papier trockengewischt und zum Abschluß mit ca. 1 ml n-Heptan abgespült.
4.) Erneutes Auswaschen des Öls und Einlegen in Altöl (analog 2, 3).

### Massensensitive Messungen:

Die Reaktionsmischung wird auf den Schwingquarz aufgebracht. Um eine konstante Schichtdicke über den gesamten, kreisförmigen Elektrodenbereich zu erzielen, verwendet man das sogenannte Spin-coating-Verfahren, bei dem der Sensor während der Polymerisation mit 200 - 400 U/min (je nach Viskosität des Schichtmaterials) rotiert. Die Schichtdicke beträgt ca. 1.5 µm, was einen Frequenzhub von ungefähr 75 kHz erzeugt. Die massensensitive Messung erfolgt mit einem Netzwerkanalysator, wodurch an das beschichtete Quarzbauteil eine Hochfrequenz variabler Frequenz angelegt wird und so das Dämpfungsverhalten des Bauteils ermittelt wird. Dies geschieht im Resonanzbereich der Dickenscherschwingung des Quarzes. Die Frequenz geringster Dämpfung wird dann vom Computer ausgelesen und als Funktion der Zeit dargestellt. Zu Beginn der Messung wird der Sensor in thermostatiertes Neuöl eingetaucht (T = 50 ± 0.1 °C). Zur schnelleren Einstellung des Sensoreffektes werden die Ölproben mit Hilfe eines Tauchrührers mit ca. 700 U/min durchmischt. Dann wird nach der Konstanz des Sensorsignals das Meßöl gewechselt. Das verwendete Altöl ist ebenfalls vorthermostatiert. Die Frequenz geringster Dämpfung (Resonanzfrequenz) ändert sich dann aufgrund zweier gegenläufiger Effekte. Zunächst induziert der Viskositätsanstieg (bei viskosem Altöl) eine Frequenzemiedrigung, die - bei chemisch sensitiver Beschichtung - teilweise durch einen Masseneffekt kompensiert wird. Letzterer wird durch das Herausdiffundieren von zuvor in die Schichten eingelagerten Molekülen erzeugt. Beim unbeschichteten oder nicht-sensitiv beschichteten Quarz beträgt der Viskositätseffekt 16 000 Hz, bei den sensitiv beschichteten Bauteilen variiert der Frequenzhub je nach Polymerschicht zwischen 11 000 und 15 000 Hz. Der Masseneffekt beträgt also je nach verwendeter Schicht 1 000 bis 5 000 Hz.

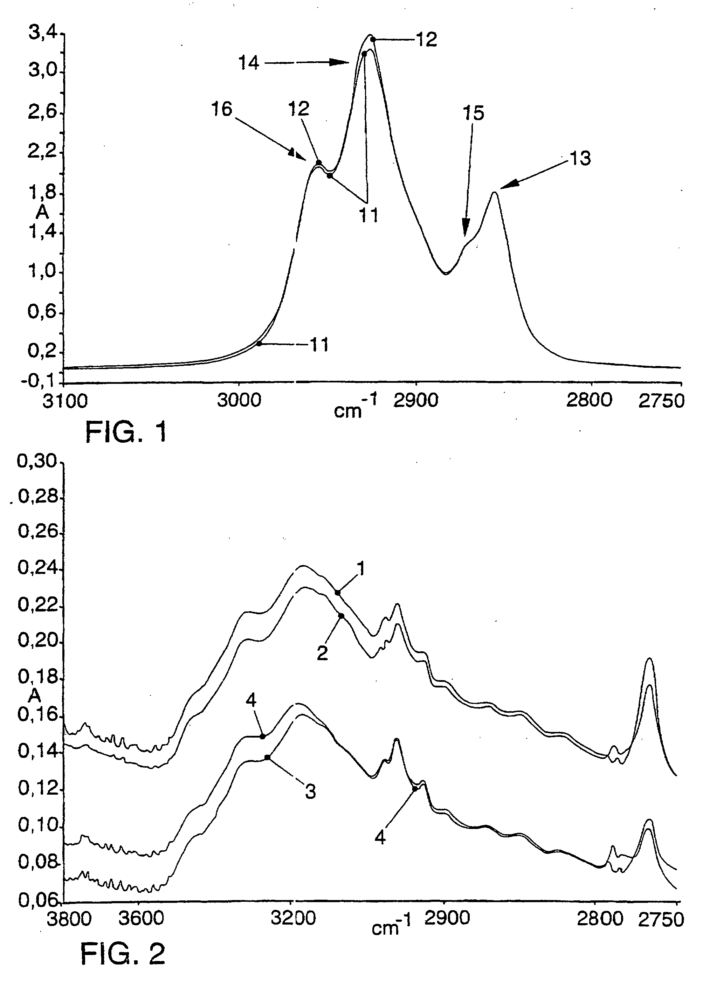

Wie aus Figur 1 ersichtlich ist, unterscheiden sich die IR-Spektren von frischem (11) und verbrauchtem (12) Otto-Motorenöl im interessierenden aliphatischen Bereich praktisch nicht. Die intensitäten der symmetrischen (13) und asymmetrischen (14) Methylenschwingungen sind nahezu identisch, auch im Bereich der Methylschwingungen (symmetrisch 15, asymmetrisch 16) sind praktisch keine Unterschiede zu erkennen.

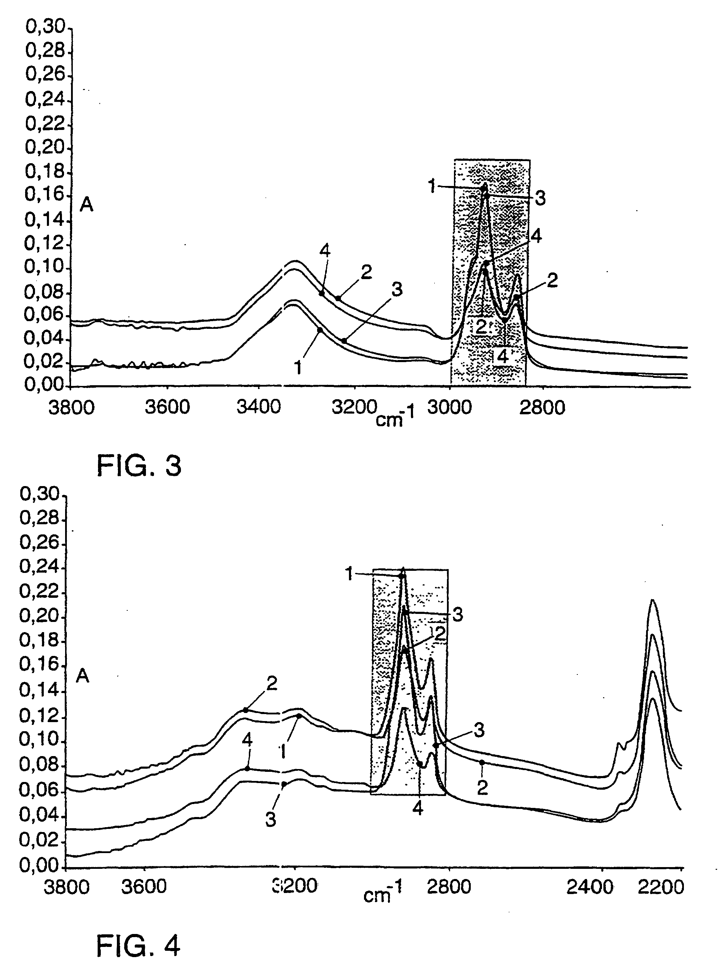

Die Figuren 2 bis 4 zeigen die jeweils nach den einzelnen Arbeitsschritten aufgenommenen IR-Spektren an drei Polymeren. Einem ohne Prägung hergestellten Polymer (Figur 2) wird ein mit frischem Öl geprägtes Polymer aus identischem Material (Figur 3) und ein mit frischem Öl geprägtes Amin-Polymer (Figur 4) gegenübergestellt. Die IR-Spektren werden nach folgenden Prozeßschritten durchgeführt:
1. Aushärten des Polymers (in Figur 3 und 4 incl. eines Otto-Neuöls);
2. Ausspülen des mobilen Anteils des zum Prägen verwendeten Imprints (Otto-Neuöl) mit n-Heptan;
3. Zwölfstündige Lagerung des Polymer in Otto-Neuöl mit anschließendem Abspülen der Oberflächenschichten; und
4. Zwölfstündige Lagerung in Otto-Altöl mit anschließendem Abspülen der Oberflächenschichten.

Bei den Proben mit geprägten Sensorschichten (Figur 3 und 4) nehmen die Intensitäten der aliphatischen Peaks (um 2 900 cm⁻¹, unterlegt) beim Ausspülen des Imprints ab, ein Großteil des Öls läßt sich also aus der polymeren Matrix entfernen. Durch das Lagern in Neuöl steigen die Intensitäten annähernd wieder auf den Ausgangswert an, das Neuöl lagert sich also wieder in die Polymermatrix ein (gleiche Spektren werden bei einer wiederholten Ein- und Auslagerung des Neuöls erhalten). Die Spektren zeigen ferner, daß Altöl (4) in das Polymergerüst nicht eingelagert wird, das IR-Spektrum entspricht dem der mit n-Heptan ausgespülten Probe.

Bei den Proben ohne Imprint (Figur 2) ergeben sich lediglich geringe Intensitätsveränderungen während der Arbeitsschritte 1 bis 4, so daß man ausschließen kann, daß es sich bei den geprägten Proben um eine reine Oberflächenadsorption handelt. Die hier geringen Effekte lassen auf eine geringfügige Porosität der Non-Imprint-Proben in Oberflächennähe schließen. Bei dem Polymer handelt es sich hier um eine reine Polyurethanschicht. Das relevante Signal liegt bei etwa 2 900 Wellenzahlen (die breiten Banden bei ca. 3 200 cm⁻¹ stellen hauptsächlich Schwingungen acider Wasserstoffe dar). Ganz anders verhält sich das gleiche Material, wenn es zusammen mit frischem Öl polymerisiert wird (Figur 3). Man sieht deutlich den Intensititätsverlust der aliphatischen CH-Schwingungen nach dem Spülen der Schicht mit n-Heptan (1. → 2.), was auf das Ausspülen des bei der Polymerisation zugegebenen Öls zurückzuführen ist. Legt man die gespülte Schicht über Nacht in Neuöl, erreicht in diesem Fall das Signal nahezu wieder seinen Ausgangswert (2. → 3.), es wird also das Neuöl wieder eingelagert. Erneutes Spülen und Einlegen in Altöl ergibt die gleiche Signalintensität wie die gespülte Schicht (2. → 4.), d. h., daß das Altöl im Gegensatz zu dem Neuöl nicht in die Schicht eingelagert wurde.

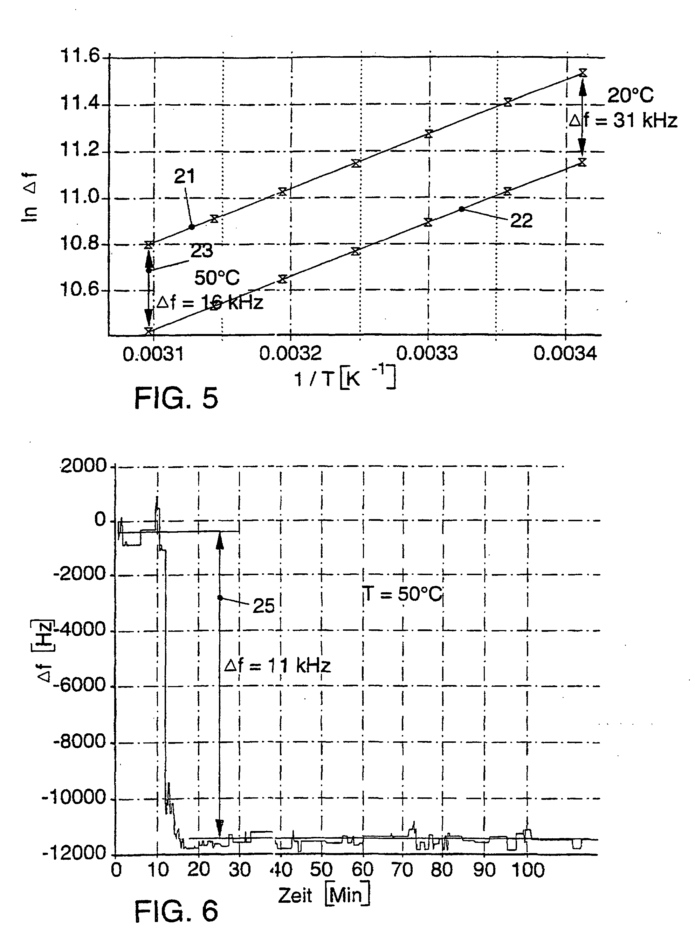

Die Figur 5 zeigt die Temperaturabhängigkeit der Resonanzfrequenz einer unbeschichteten QMB in Altöl 22 bzw. in Neuöl 21. Bei 50 °C beträgt der Frequenzhub 23 der in das jeweilige Öl getauchten Quarze 16 kHz beim Übergang von Neuöl in Altöl. Ähnliche Werte werden mit Quarzen erhalten, die mit Polymeren beschichtet sind, die kein Öl inkorparieren können (ungeprägte Polymere, Non-Imprint-Probe). Es kann ein linearer Zusammenhang zwischen dem Logarithmus des Frequenzhubes und der reziproken absoluten Temperatur beobachtet werden. Schwingquarze mit nicht-sensitiven Polymerschichten (Non-Imprint) ergeben parallel verschobene Geraden, deren Steigungen sich maximal um 10 % unterscheiden. Daraus folgt, daß es sich bei den gemessenen Frequenzhüben um reine Viskositätseffekte und nicht um Masseneffekte handelt.

Der Frequenzhub, der beim Transfer einer mit einer durch molekulares Prägen hergestellten sensitiven Schicht (Imprint-Probe) beladenen QMB (Quarz Micro Balance) von Neuöl in Altöl beobachtet werden kann, liegt bei einer Temperatur von 50 °C anfänglich um 2 bis 3 kHz niedriger als für einen Non-Imprint-Quarz. Je nach Polymerschicht kann der Frequenzhub auch noch geringer ausfallen. Die Figur 6 zeigt einen Frequenzhub 25 von 11 kHz für einen Quarz mit einer sensitiven Schicht, d. h., dieser Frequenzhub 25 liegt 5,0 kHz unter dem eines unbeschichteten Quarzes (auch ein Quarz mit einer Non-Imprint-Schicht ist hier einsetzbar). Dieser Unterschied in der Frequenzantwort einer sensitiv beschichteten QMB zu allen anderen Quarzen resultiert aus der reversiblen Einlagerung von Otto-Neuöl in die Schicht und ein Herauslösen des Otto-Neuöls aus der Schicht, wenn diese in Altöl getaucht wird. Der Frequenzhub für unterschiedliche nicht-sensitive Schichten ist näherungsweise gleich. Dieser Sachverhalt ist in der Figur 7 näher dargestellt.

Der nicht mit einer sensitiven Schicht versehene Quarz reagiert nahezu ausschließlich auf die unterschiedliche Viskosität von Neuöl 31 und Altöl 32, ohne Berücksichtigung deren chemischer Zusammensetzungen. Die Frequenzantwort Δf beträgt 16 kHz. Bei der parallel durchgeführten Messung des Frequenzhubs an dem mit einer sensitiven Schicht versehenen Quarz wird (s. o.) lediglich ein Betrag von 11 bis 14 kHz (je nach sensitiver Schicht) erhalten, d. h. ca. 2 bis 5 kHz weniger. Dieser Betrag resultiert aus dem Masseneffekt, d. h. dem Gewichtsunterschied der mit Neuöl 33 beladenen sensitiven Schicht und dem Herauslösen des Neuöls 33 aus der sensitiven Schicht, wenn diese in Altöl 32 lagert.

Mit der Verwendung eines Quarzes mit nicht-sensitiver Schicht (oder ohne Beschichtung) als Referenz kann also zum einen die Viskositätsveränderung von Neuöl zu Altöl und der Verbrauch des Neuöls (Alterung) bestimmt werden. Letzterer verläuft proportional zum Masseneffekt Δm. Mit dem erfindungsgemäßen Sensor läßt sich entsprechend die Alterung eines Motorenöls fortlaufend durch Zunahme des Masseneffekts überwachen, wobei die Veränderung der Viskosität, die von einer Zunahme bis hin zu einer Abnahme reicht, durch die Referenz berücksichtigt wird.

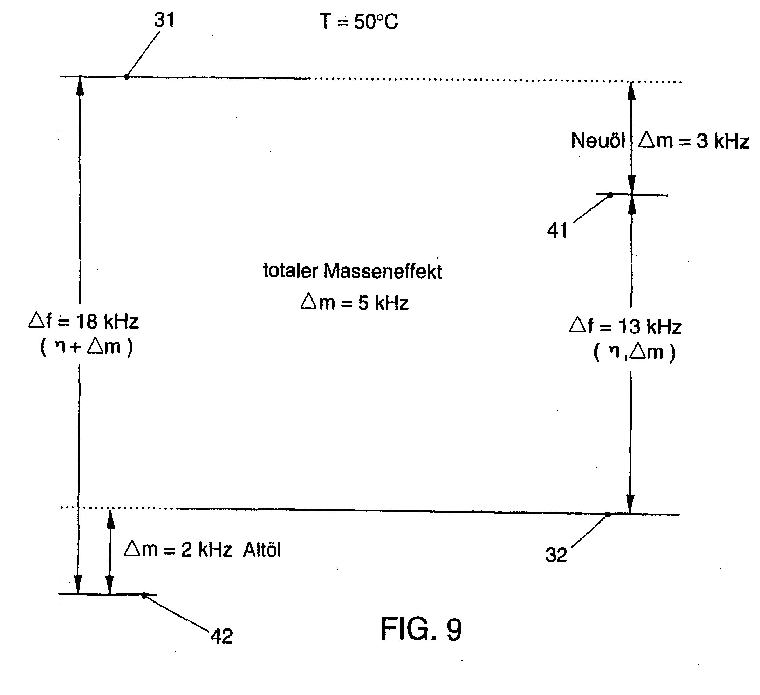

Alternativ oder zusätzlich zu dem auf Neuöl sensitiven oder nicht sensitiven Quarz kann auch ein auf Altöl sensitiver Quarz eingesetzt werden. Die Figur 9 zeigt den Vergleich zwischen einer mit Altöl geprägten Quarzbeschichtung 42 und einer mit Neuöl geprägten Quarzbeschichtung 41. Der auf Neuöl geprägte Quarz 41 zeigt in Neuöl eine Frequenzshift von 16 kHz, die in Altöl auf 13 kHz abnimmt. Hingegen zeigt der auf Altöl geprägte Quarz 42 in frischem Öl eine Frequenzshift von 16 kHz, die in Altöl auf 18 kHz zunimmt.

Durch die Verwendung von Triethanolamin können vermutlich auch Wasserstoff-Brückenbindungen zur Bindung der aciclen Komponenten des Altöls herangezogen werden. Die Ausnützung dieser polaren Wechselwirkungen bietet das Potential für die Entwicklung eines Sensors für jedes beliebige Motorenöl, da durch Verbrennungsprozesse immer Carboxylgruppen (Säuregruppen) gebildet werden. Mit Polymerbausteinen unterschiedlicher Basizität können den einzelnen aciden Komponenten des Altöls angepaßte Wechselwirkungen erreicht werden. Der Altölsensor ist auch als Schwellwertsensor auslegbar, der bei einer vorgegebenen Acidität anspricht und hierdurch einen Ölwechsel signalisiert. Mit der Acidität kann eine Eigenschaft des Öls erfaßt werden, die unabhängig von der Marke und Qualität der Öle ist, so daß auch synthetische Öle hinsichtlich ihrer Alterung überwacht werden können.

Erfindungsgemäß erfolgt die Ölqualitätsbestimmung vorteilhaft also mittels eines mit einer sensitiven Schicht beschichteten Quarzes. Die sensitive Schicht hat eine an mindestens einen Ölbestandteil adaptierte Oberfläche, die entsprechend der Konzentration des Ölbestandteils zur wiederholten Ein- und Auslagerung des Ölbestandteils prädestiniert ist. Beim Vorliegen des Ölbestandteils lagert sich dieser in die sensitive Schicht ein, wodurch über einen Masseneffekt, eine effektive Steigerung der Bauteildicke bzw. -masse, die Resonanzfrequenz sinkt. Durch Alterung des Öls nimmt der Bestandteil, der in die sensitive Schicht einlagert ab, wodurch die Resonanzfrequenz ansteigt. Als Referenz wird eine nicht-sensitive Schicht verwendet, über die der Viskositätseffekt des Öls auf die Schwingung des Quarzes ermittelt wird.

## Patentansprüche

1. Verwendung eines Sensors mit einer sensitiven Schicht, die eine an mindestens einen Analyten adaptierte Oberfläche und/oder der ein an mindestens einen Analyten adaptiertes Schichtvolumen hat, die bzw. das entsprechend einer vorliegenden Affinität des Analyten zu der Schicht zur analytspezifischen wiederholten Ein- und Auslagerung des Analyten prädestiniert ist, zur Feststellung des Analyten in einem Öl und/oder zur Feststellung eines öligen Analyten, wobei der Sensor zur Feststellung einer Alterung des Öls bestimmt ist und die sensitive Schicht zusammen mit dem Analyten gebildet ist beziehungsweise mit dem Analyten geprägt ist,
wobei die Feststellung des Analyten in dem Öl und/oder des öligen Analyten durch Ermittlung einer Gewichtsveränderung der sensitiven Schicht erfolgt.

2. Verwendung nach Anspruch 1, **dadurch gekennzeichnet, dass** das Öl beziehungsweise der ölige Analyt ein Motorenöl, insbesondere für eine Brennkraftmaschine ist.

3. Verwendung nach einem der Ansprüche 1 oder 2, **dadurch gekennzeichnet, dass** das Material beziehungsweise die sensitive Schicht ein Polymer ist.

4. Verwendung nach Anspruch 3, **dadurch gekennzeichnet, dass** das Polymer ein vernetztes Polyurethan und/oder ein derivatisiertes Polyurethan ist.

5. Verwendung nach Anspruch 3 oder 4, **dadurch gekennzeichnet, dass** das Polymer basische Gruppen, insbesondere Aminogruppen enthält.

6. Verwendung nach einem der Ansprüche 2 bis 5, **dadurch gekennzeichnet, dass** das Polymer in Gegenwart von Altöl gebildet ist bzw. mit Altöl geprägt ist.

7. Verwendung nach einem der vorhergehenden Ansprüche, **dadurch gekennzeichnet, dass** der Analyt inert zur sensitiven Schicht ist.

8. Verwendung nach einem der vorhergehenden Ansprüche, **dadurch gekennzeichnet, dass** der Sensor neben der sensitiven Schicht noch eine nicht-sensitive Referenz enthält.

9. Verwendung nach Anspruch 8, **dadurch gekennzeichnet, dass** der Sensor neben dem Analyt auch eine weitere Größe, insbesondere eine Eigenschaft der Flüssigkeit, insbesondere die Viskosität, erfasst.

10. Verwendung nach einem der vorhergehenden Ansprüche, **dadurch gekennzeichnet, dass** die Ein- bzw. Auslagerung des Analyten in bzw. aus der sensitiven Schicht durch Bestimmung der Massenveränderung der sensitiven Schicht, insbesondere durch Schwingen der sensitiven Schicht, bestimmt wird.

11. Verwendung nach einem der vorhergehenden Ansprüche, **dadurch gekennzeichnet, dass** die sensitive Schicht eine Matrix mit an den Analyt adaptierten Cavitäten und/oder Diffusionskanälen ist.

12. Ölqualitätssensor, insbesondere für eine Brennkraftmaschine, mit einer sensitiven Schicht, die eine speziell an mindestens einen in einem Öl vorliegenden Bestandteil, dessen Affinität zu der Schicht sich beim vorgesehenen Gebrauch des Öls ändert, adaptierte Oberfläche oder Schichtvolumen hat, die bzw. das entsprechend einer vorliegenden Affinität des Bestandteils zu der Schicht zur spezifischen Wiedereinlagerung des Bestandteils prädestiniert ist, wobei der Sensor zur Feststellung einer Alterung des Öls bestimmt ist und die sensitive Schicht zusammen mit dem im Öl vorliegenden Bestandteil gebildet ist,
wobei der Ölqualitätssensor bezüglich der sensitiven Schicht massesensitiv ist.

13. Ölqualitätssensor nach Anspruch 12, **dadurch gekennzeichnet, dass** die sensitive Schicht eine Polymerschicht ist.

14. Ölqualitätssensor gemäß einem der Ansprüche 12 oder 13, **dadurch gekennzeichnet, dass** er neben der sensitiven Schicht eine nicht-sensitive Referenz enthält.

15. Verfahren zur Feststellung eines Analyten in einer flüssigen Phase mittels eines Sensors mit einer sensitiven Schicht, die eine an mindestens einen Analyt adaptierte Oberfläche und/oder die ein an mindestens einen Analyt adaptiertes Schichtvolumen hat, die bzw. das entsprechend einer vorliegenden Affinität des Analyten zu der Schicht zur analytspezifischen wiederholten Ein- und Auslagerung des Analyten prädestiniert ist, wobei die Feststellung des Analyten in der flüssigen Phase durch Ermittlung einer Gewichtsveränderung der sensitiven Schicht erfolgt, wobei der Sensor zur Feststellung einer Alterung eines Öls bestimmt ist und die sensitive Schicht zusammen mit dem Analyten gebildet ist, beziehungsweise mit dem Analyten geprägt ist.

16. Verfahren nach Anspruch 15, **dadurch gekennzeichnet, dass** eine Motorenölalterung festgestellt wird.

## Claims

1. Use of a sensor having a sensitive layer which has a surface adapted to at least one analyte and/or which has a layer volume adapted to at least one analyte, said surface or layer volume being preorganized for the analyte-specific repeated incorporation and release of the analyte in accordance with an existing affinity of the analyte for the layer, for the detection of the analyte in an oil and/or for the detection of an oily analyte, the sensor being intended for the detection of ageing of the oil and the sensitive layer having been formed together with the analyte or having been imprinted with the analyte,
in which the analyte in the oil and/or the oily analyte is/are detected by determination of a change in the weight of the sensitive layer.

2. Use according to Claim 1, **characterized in that** the oil or the oily analyte is a motor oil, in particular for an internal combustion engine.

3. Use according to one of Claims 1 and 2, **characterized in that** the material or the sensitive layer is a polymer.

4. Use according to Claim 3, **characterized in that** the polymer is a crosslinked polyurethane and/or a derivatized polyurethane.

5. Use according to Claim 3 or 4, **characterized in that** the polymer contains basic groups, in particular amino groups.

6. Use according to one of Claims 2 to 5, **characterized in that** the polymer has been formed in the presence of used oil or has been imprinted with used oil.

7. Use according to one of the preceding claims, **characterized in that** the analyte is inert toward the sensitive layer.

8. Use according to one of the preceding claims, **characterized in that** the sensor, in addition to the sensitive layer, also comprises a non-sensitive reference.

9. Use according to Claim 8, **characterized in that** the sensor, in addition to the analyte, also measures a further parameter, in particular a property of the liquid, in particular the viscosity.

10. Use according to one of the preceding claims, **characterized in that** the incorporation and release of the analyte into and out of the sensitive layer is determined by determining the change in mass of the sensitive layer, in particular by oscillation of the sensitive layer.

11. Use according to one of the preceding claims, **characterized in that** the sensitive layer is a matrix having cavities and/or diffusion channels adapted to the analyte.

12. Oil quality sensor, especially for an internal combustion engine, having a sensitive layer which has a surface or layer volume which is adapted specifically to at least one constituent present in an oil, the affinity of said constituent for the layer changing in the course of the intended use of the oil, said surface or layer volume being preorganized for the specific reincorporation of the constituent in accordance with an existing affinity of the constituent for the layer, the sensor being intended for the detection of ageing of the oil and the sensitive layer being formed together with the constituent present in the oil, in which the oil quality sensor is mass-sensitive with respect to the sensitive layer.

13. Oil quality sensor according to Claim 12, **characterized in that** the sensitive layer is a polymer layer.

14. Oil quality sensor according to one of Claims 12 and 13, **characterized in that** it comprises, in addition to the sensitive layer, a non-sensitive reference.

15. Method for detecting an analyte in a liquid phase by means of a sensor having a sensitive layer which has a surface adapted to at least one analyte and/or which has a layer volume adapted to at least one analyte, said surface or layer volume being preorganized for the analyte-specific repeated incorporation and release of the analyte in accordance with an existing affinity of the analyte for the layer, the analyte in the liquid phase being detected by determination of a change in weight of the sensitive layer, the sensor being intended for the detection of ageing of an oil and the sensitive layer having been formed together with the analyte or having been imprinted with the analyte.

16. Method according to Claim 15, **characterized in that** ageing of a motor oil is detected.

## Revendications

1. Utilisation d'un détecteur qui présente une couche sensible dont la surface et/ou le volume sont adaptés à au moins un analyte et qui sert à l'adsorption et à la désorption répétées de l'analyte de façon spécifique à celui-ci et selon son affinité vis-à-vis de la couche afin de déterminer l'analyte dans une huile et/ou de déterminer un analyte huileux, le détecteur servant à déterminer le vieillissement de l'huile et la couche sensible étant formée avec l'analyte ou étant imprégnée de celui-ci, dans lequel l'analyte présent dans l'huile et/ou l'analyte huileux sont déterminés à partir de la modification du poids de la couche sensible.

2. Utilisation selon la revendication 1, **caractérisée en ce que** l'huile ou l'analyte huileux est une huile de moteur et en particulier d'un moteur à combustion interne.

3. Utilisation selon l'une des revendications 1 ou 2, **caractérisée en ce que** le matériau et la couche sensible sont des polymères.

4. Utilisation selon la revendication 3, **caractérisée en ce que** le polymère est un polyuréthane réticulé et/ou un dérivé de polyuréthane.

5. Utilisation selon les revendications 3 ou 4, **caractérisée en ce que** le polymère contient des groupes basiques et en particulier des groupes amine.

6. Utilisation selon l'une des revendications 2 à 5, **caractérisée en ce que** le polymère est formé en présence d'huile usée ou en est imprégné.

7. Utilisation selon l'une des revendications précédentes, **caractérisée en ce que** l'analyte ne réagit pas avec la couche sensible.

8. Utilisation selon l'une des revendications précédentes, **caractérisée en ce qu'**outre la couche sensible, le détecteur présente une référence non sensible.

9. Utilisation selon la revendication 8, **caractérisée en ce qu'**outre l'analyte, le détecteur saisit également une autre grandeur, en particulier une propriété du liquide et en particulier sa viscosité.

10. Utilisation selon l'une des revendications précédentes, **caractérisée en ce que** l'adsorption de l'analyte par la couche sensible ou sa désorption sont déterminées à partir de la modification de la masse de la couche sensible et en particulier mettant la couche sensible en oscillation.

11. Utilisation selon l'une des revendications précédentes, **caractérisée en ce que** la couche sensible est une matrice qui présente des cavités et/ou des canaux de diffusion adaptés à l'analyte.

12. Détecteur de qualité d'huile, en particulier pour un moteur à combustion interne, qui présente une couche sensible dont la surface ou le volume sont spécialement adaptés à un composant présent dans une huile et dont l'affinité varie lors de l'usage prévu de l'huile et qui est destinée à réadsorber le composant de manière spécifique à celui-ci et selon son affinité vis-à-vis de la couche, le détecteur servant à déterminer le vieillissement de l'huile et la couche sensible étant formée avec le composant présent dans l'huile, le détecteur de qualité d'huile étant sensible à la masse de la couche sensible.

13. Détecteur de qualité d'huile selon la revendication 12, **caractérisé en ce que** la couche sensible est une couche polymère.

14. Détecteur de qualité d'huile selon l'une des revendications 12 ou 13, **caractérisé en ce qu'**outre la couche sensible, il présente une référence non sensible.

15. Procédé de détermination d'un analyte dans une phase liquide au moyen d'un détecteur qui présente une couche sensible dont la surface et/ou le volume sont adaptés à au moins un analyte et qui est destinée à l'adsorption et à la désorption répétées de l'analyte, de façon spécifique à celui-ci et selon son affinité à la couche, la détermination de l'analyte dans la phase liquide étant réalisée par l'intermédiaire de la saisie d'une modification de poids de la couche sensible, dans lequel la couche sensible est formée avec l'analyte ou en est imprégnée.

16. Procédé selon la revendication 15, **caractérisé en ce qu'**il détermine le vieillissement d'une huile de moteur.
